# EUROPEAN PATENT SPECIFICATION

(11) **EP 3 695 004 B1**
(45) Date of publication and mention of the grant of the patent: **12.11.2025**
(21) Application number: 18792601.9
(22) Date of filing: 21.09.2018
(51) Int. Cl.: C12Q 1/22, C12M 1/34, C12M 1/12, A61L 2/28

(54) **BIOLOGICAL INDICATOR**
BIOLOGISCHER INDIKATOR
INDICATEUR BIOLOGIQUE

(30) Priority: 11.10.2017 US 201715729685
(43) Date of publication of application: 19.08.2020
(60) Divisional application: 25197708.8
(73) Proprietor: American Sterilizer Company, Mentor, OH 44060 (US)
(72) Inventor: CREGGER, Tricia, Fairlawn, Ohio 44333 (US); YIRAVA, William, Willoughby, Ohio 44094 (US); FRANCISKOVICH, Phillip P., Concord, Ohio 44077 (US); FIX, Kathleen A., Willoughby, Ohio 44094 (US)
(74) Representative: Brand Murray Fuller LLP
(86) International application number: PCT/US2018/052099
(87) International publication number: WO 2019/074642

(56) References cited:
- WO-A1-2017/131819
- MARINA RAGUSE ET AL: "Improvement of Biological Indicators by Uniformly Distributing Bacillus subtilis Spores in Monolayers To Evaluate Enhanced Spore Decontamination Technologies", APPLIED AND ENVIRONMENTAL MICROBIOLOGY, vol. 82, no. 7, 22 January 2016 (2016-01-22), US, pages 2031 - 2038, XP055526049, ISSN: 0099-2240, DOI: 10.1128/AEM.03934-15

## Description

### Technical Field

This invention relates to a biological indicator for monitoring the effectiveness of a sterilization process, a sterilization process and a process for determining the effectiveness of a sterilization process.

### Background

Biological indicators, which typically comprise a carrier and test microorganisms (e.g., bacterial spores) deposited on the carrier, are used to monitor the effectiveness of sterilization processes. The biological indicator is placed in a sterilization chamber and subjected to a sterilization cycle along with the load intended for sterilization (e.g., a medical device). Following the sterilization cycle, the biological indicator is exposed to a growth media and incubated for the purpose of determining if any of the test microorganisms are viable. A successful sterilization cycle is indicated by a complete inactivation (no outgrowth) of the test microorganisms. An unsuccessful sterilization cycle is indicated by an incomplete inactivation (outgrowth detected) of the test microorganisms.

Raguse et al: "Improvement of Biological Indicators by Uniformly Distributing Bacillus subtilis Spores in Monolayers to Evaluate Enhanced Spore Decontamination Technologies", Applied and Environmental Microbiology, Vol. 82, No. 7, 22 January 2016, pages 2031-2028 relates to an electrically driven spray deposition device for the preparation of spore monolayers.

WO 2017131819 relates to a biological indicator comprising a carrier inoculated with a test organism and an effective amount of a carbohydrate to reduce the resistance of the biological indicator to steam sterilization.

### Summary

This invention relates to a biological indicator, comprising: a carrier; and a spore deposit on the carrier, wherein from about 70% to about 95% of the area of the spore deposit comprises spores residing in a single spore layer, and the remainder of the area of the spore deposit comprises spores residing in a stacked configuration, and wherein the biological indicator is in a first compartment of an indicator device, the first compartment being adapted to permit the spores to be brought into contact with a sterilant during a sterilization process; and the indicator device further comprises a second compartment containing a growth media, the second compartment being adapted to maintain the growth media separate from the spores during the sterilization process, and to permit the growth media to contact the spores after the sterilization process is completed.

A problem in the art relates to the fact that biological indicators are typically designed to be sterile prior to the end of a sterilization cycle. That is, they are designed in such a manner that for a successful sterilization all of the spores on the biological indicator will be killed prior to the end of the sterilization cycle. In some cases, the biological indicators are designed not to exceed the half cycle (i.e., half way through a typical exposure phase during a sterilization cycle). Many biological indicators become sterile much earlier than at the half cycle due in part to significant spore kill in pre-exposure conditioning phases. For this reason, there are often significant portions of the exposure phase during a sterilization cycle that are not monitored by live spores. This can lead to problems in determining whether a sterilization is successful.

One solution to this problem involves increasing the population bioload of the spores on the carrier. This can be achieved by depositing an inoculum containing a high concentration of the spores on the carrier. This can result in a significant delay until all the spores are killed. A problem with this approach, however, relates to the fact that this often leads to uncontrolled spore stacking. Spore stacking occurs when spores overlie one another such that spores on top protect spores underneath from exposure to a sterilant during a sterilization cycle. Too much spore stacking can lead to false positives where some of the underlying spores survive the full cycle of a sterilization process even though conditions for a successful sterilization are met.

This invention provides a solution to this problem. With this invention it is possible to combine sufficient resistance to extend coverage further into the exposure phase of a sterilization cycle and simultaneously ensure that the spores do not survive the end of a successful sterilization cycle due to excess stacking. This invention involves depositing spores on a carrier in a way in which spore stacking is controlled within predetermined limits to allow the resulting biological indicator to provide coverage throughout a substantial part (e.g., at least about 25%, or at least about 33%, or at least about 50%, or at least about 67%, or at least about 75%, or at least about 90%, or at least about 95%) of the sterilization cycle, but not survive the end of a successful sterilization cycle.

In a first aspect, this invention relates to a biological indicator, comprising: a carrier; and a spore deposit on the carrier, wherein from about 70% to about 95%, optionally from about 80% to about 95%, or from about 90% to about 95%, of the area of the spore deposit comprises spores residing in a single spore layer, and the remainder of the area of the spore deposit comprises spores residing in a stacked configuration and wherein the biological indicator is in a first compartment of an indicator device, the first compartment being adapted to permit the spores to be brought into contact with a sterilant during a sterilization process; and the indicator device further comprises a second compartment containing a growth media, the second compartment being adapted to maintain the growth media separate from the spores during the sterilization process, and to permit the growth media to contact the spores after the sterilization process is completed. In an embodiment, from about 5% to about 30%, or from about 5% to about 20%, or from about 5 to about 10%, of the area of the spore deposit comprises spores residing in a stacked configuration. The spore deposit may have a total number of spores in the range from about 1 x 10⁶ to about 1 x 10⁸ colony forming units (cfu), or from about 1 x 10⁷ to about 1 x 10⁸ cfu. In an embodiment, the spore deposit is formed by the deposition of an inoculum (e.g., one or more drops of an inoculum, or from 1 to about 10, or from 2 to about 10, or from 2 to about 8, or from 2 to about 6, or from 2 to about 4, or 2 or 3 drops of an inoculum) on the carrier. In an embodiment, the inoculum comprises water and the spores dispersed in the water. In an embodiment, the inoculum is dried on the carrier to form the spore deposit. In an embodiment, the inoculum deposited on the carrier forms a perimeter upon being deposited on the carrier, and prior to being dried the inoculum is spread to extend the perimeter. The concentration of spores in the inoculum may be in the range from about 1 x 10³ to about 1 x 10⁶ cfu per microliter, or from about 1 x 10⁴ to about 1 x 10⁵ cfu per microliter. In an embodiment, the carrier comprises a hydrophobic substrate. The carrier may comprise metal, glass, ceramics, plastic, or a combination of two or more thereof. The water may comprise tap water, deionized water, distilled water, water purified by osmosis, or a mixture of two or more thereof. In an embodiment, the inoculum deposited on the carrier has a volume in the range from about 10 to about 1000 microliters, or from about 10 to about 500 microliters, or from about 10 to about 50 microliters.

The biological indicator is positioned in a first compartment of an indicator device, the first compartment being adapted to permit the spores to be brought into contact with a sterilant during a sterilization process. The indicator device further comprises a second compartment containing a growth media, the second compartment being adapted to maintain the growth media separate from the spores during the sterilization process, and the second compartment being adapted to permit the growth media to contact the spores after the sterilization process is completed.

The indicator device may be referred to as a self-contained biological indicator. **In** an embodiment, the indicator device is positioned in a test pack.

In an embodiment, the spores comprise bacterial spores. In an embodiment, the spores comprise spores of the *Bacillus* or *Clostridia* genera. In an embodiment, the spores comprise spores of *Geobacillus stearothermophilus, Bacillus atrophaeus, Bacillus sphaericus, Bacillus anthracis, Bacillus pumilus, Bacillus coagulans, Clostridium sporogenes, Clostridium difficile, Clostridium botulinum, Bacillus subtilis globigii, Bacillus cereus, Bacillus circulans,* or a mixture of two or more thereof. In an embodiment, the spores may comprise *Geobacillus stearothermophilus* spores.

In a second aspect, this invention relates to a sterilization process, comprising: exposing an article to be sterilized and the above-indicated biological indicator to a sterilant. In an embodiment, the sterilant comprises vaporous hydrogen peroxide, ethylene oxide, steam, peracetic acid, oxone, or a combination of two or more thereof.

In a third aspect, this invention relates to a process for determining the effectiveness of a sterilization process, comprising: exposing an article to be sterilized and the above-indicated biological indicator to a sterilant; and incubating the biological indicator in the presence of a growth media to determine whether the sterilization process is effective.

Disclosed herein, but not claimed, is a process for making a biological indicator, comprising: (A) forming an inoculum comprising water and spores, the concentration of the spores in the inoculum being in the range from about 1 x 10³ to about 1 x 10⁶ colony forming units per microliter; (B) depositing the inoculum on a carrier, the volume of the inoculum being deposited on the carrier being in the range from about 10 to about 1000 microliters; and (C) drying the inoculum to form a spore deposit on the carrier, wherein from about 70% to about 95%, or from about 80% to about 95%, or from about 90% to about 95%, of the area of the spore deposit comprises spores residing in a single spore layer, with the remainder of the spore deposit comprising spores residing in a stacked configuration.

### Brief Description of the Drawings

Fig. 1 is a perspective view of an indicator device in the form of a self-contained biological indicator (SCBI) which can be used in accordance with the present invention.
Fig. 2 is a cross-sectional view of the SCBI of Fig. 1 (taken along the line 2-2 in Fig. 1) showing a cap mounted on a container in a first non-activated position.
Fig. 3 is a cross-sectional view of the SCBI of Fig. 1 (taken along the line 2-2 in Fig. 1) showing the cap mounted on the container in a second activated position.
Fig. 4 is a cross-sectional view of the SCBI of Fig. 1 (taken along line 4-4 in Fig. 1) showing the indicator in the second activated position.
Fig. 5 is a side-top perspective view of a test pack that can be used with the SCBI shown in Figs. 1-4.
Fig. 6 is a cross-sectional view of the test pack illustrated in Fig. 5 taken along lines 6-6 of Fig. 5.
Fig. 7 is a top plan view of a test pack that can be used in accordance with the present invention, with an SCBI in a first recessed compartment and a chemical integrator and/or chemical indicator in a second recessed compartment.
Fig. 8 is a schematic illustration of spore deposits from (A) a 20 microliter drop of an inoculum and (B) a 40 microliter drop of an inoculum. These spore deposits are described in Examples 1 and 2.
Fig. 9 is a low magnification photomicrograph of the 20 microliter drop (A) and the 40 microliter drop (B) described in Examples 1 and 2.
Fig. 10 is a 40X magnification photomicrograph of a spore deposit from the 20 microliter drop (A) described in Examples 1 and 2.
Fig. 11 is a 40X magnification photomicrograph of a spore deposit from the 40 microliter drop (B) described in Examples 1 and 2.
Fig. 12 is a 200X magnification photomicrograph of a spore deposit from the 20 microliter drop (A) described in Examples 1 and 2.
Fig. 13 is a 200X magnification photomicrograph of a spore deposit from the 40 microliter drop (B) described in Examples 1 and 2.
Fig. 14 is a low magnification photomicrograph of a spore deposit from the 10 microliter drop (C) described in Example 3.
Fig. 15 is a 40X magnification photomicrograph of the 10 microliter drop (C) described in Example 3.
Fig. 16 is a 200X magnification photomicrograph of a spore deposit from the 10 microliter drop (C) described in Example 3.

### Detailed Description

All ranges and ratio limits disclosed in the specification and claims may be combined in any manner. It is to be understood that unless specifically stated otherwise, references to "a," "an," and/or "the" may include one or more than one, and that reference to an item in the singular may also include the item in the plural.

The phrase "and/or" should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in one embodiment, to A without B (optionally including elements other than B); in another embodiment, to B without A (optionally including elements other than A); in yet another embodiment, to both A and B (optionally including other elements); etc.

The word "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," may refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

The phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

The transitional words or phrases, such as "comprising," "including," "carrying," "having," "containing," "involving," "holding," and the like, are to be understood to be open-ended, i.e., to mean including but not limited to.

The term "bacteria" refers to a domain of prokaryotic microorganisms. The bacteria may be unicellular microorganisms. The cells may be described as prokaryotic because they lack a nucleus. The bacteria cells may have one of four major shapes: bacillus (rod shaped), coccus (spherical shape), spirilla (spiral shape), or vibrio (curved shape). **The** bacteria may have a peptidoglycan wall. **The** bacteria may divide by bacteria fission. **The** bacteria may possess flagella for motility. **The** bacteria may be classified as either Gram-positive or Gram-negative when using Gram staining. **The** bacteria may be divided based on their response to gaseous oxygen into the following groups: aerobic (living in the presence of oxygen), anaerobic (living without oxygen), and facultative anaerobic (can live in both environments). **The** bacteria may be classified as heterotrophs or autotrophs. Autotrophs make their own food by using the energy of sunlight or chemical reactions, in which case they are called chemoautotrophs. Heterotrophs obtain their energy by consuming other organisms. **The** bacteria that use decaying life forms as a source of energy may be called saprophytes.

The term "spore" refers to a unit of asexual reproduction that may be adapted for dispersal and survival for extended periods of time under unfavorable conditions. Spores are highly resistant, dormant cell types. Endospores (or simply spores) form within the vegetative mother cell in response to adverse changes in the environment, most commonly nutrient depletion. The mother cell undergoes an asymmetrical cell division, where it replicates its genetic material, which is then surrounded by multiple concentric and spore specific layers. The mother cell then disintegrates, releasing the mature dormant spore which requires neither nutrients, water nor air for survival and is protected against a variety of trauma, including extremes of temperature, radiation, and chemical assault.

The term "bacterial spore" refers to a spore produced by bacteria.

The term "biological indicator" refers to an article that can be used to determine the efficacy of a sterilization process. The biological indicator of the invention comprises spores deposited on a carrier. The spores may be referred to as test microorganisms.

The term "carrier" refers to a support onto which spores are deposited to form a biological indicator.

The term "spore stacking" refers to a spore deposit wherein spores are stacked on top of one another. The spores on top tend to shield spores underneath from exposure to a sterilant during a sterilization process. Spore stacking can be detected using light microscopy and electron microscopy.

The term "single spore layer" refers to a layer of spores wherein no spore stacking occurs. A spore deposit with a single spore layer has a thickness of one spore. A single spore layer may be referred to as a monodispersed spore layer. With this invention spore deposits are used wherein from 70% to about 95%, or from about 80% to about 95%, or from about 90% to about 95% of the area of the spore deposit may comprise spores that reside in a single spore layer, with the remainder of the spore deposit comprising spores residing in a stacked configuration. From about 5% to about 30%, or from about 5% to about 20%, or from about 5% to about 10% of the area of the spore deposit may comprise spores residing in a stacked configuration.

The term "area of the spore deposit" refers to the area of a spore deposit that is measured after an inoculum containing the spores is deposited on the carrier and dried. Drying refers to sufficient drying to allow the spore deposit to be used as a biological indicator in a sterilization process. The spore deposit can be formed using an ink jet printer to deposit the inoculum on the carrier. The area of the spore deposit that is in the form of a single spore layer can be determined during production on an ink jet printer by monitoring the deposition using a fiduciary camera and knowing the concentration of the inoculum dispensed by the printer (in the nano to picoliter range), or by use of a light microscope to get overall view of the deposit, or by an electron microscope which allows resolution of individual spores.

The term "inoculated carrier" refers to a carrier onto which an inoculum containing spores has been deposited.

The term "inoculum" refers to a composition containing water and spores dispersed in the water. The inoculum can be used to inoculate a carrier. In an embodiment, the inoculum is used to form a biological indicator by depositing the inoculum on a carrier, and then drying the inoculum leaving a spore deposit on the carrier.

In an embodiment, the carrier comprises a hydrophobic substrate. The term "hydrophobic substrate" refers to a substrate that repels a drop of water such that the drop of water beads up when deposited on the substrate. In an embodiment, a drop of water deposited on the hydrophobic substrate will bead up with a contact angle greater than 90°. The contact angle may be measured using a contact angle goniometer.

The spores used with the inventive biological indicator may be used as test microorganisms during a sterilization process. Test microorganisms are typically selected from those microorganisms that are more resistant to the sterilization process being evaluated than the organisms to be killed by the sterilization process. Spores, especially bacterial spores, which are difficult to kill, are often used as test microorganisms for sterilization processes. The killing of the test microorganisms during a sterilization process is indicative of a successful sterilization process.

The term "killing" spores refers to rendering spores incapable of reproduction, metabolism and/or growth. The term "dead" spores refers to spores which have been rendered incapable of reproduction, metabolism and/or growth.

The term "live" spores refers to spores that are capable of reproduction, metabolism and/or growth.

The term "log reduction" is a mathematical term to show the number of spores used as test microorganisms that are killed by contacting the spores with a sterilant during a sterilization process. A "4 log reduction" means that the number of live spores at the end of the sterilization process is reduced by 10,000-fold. A "5 log reduction" means that the number of live spores is reduced by 100,000-fold. A "6 log reduction" means that the number of live spores is reduced by 1,000,000-fold. Thus, for example, if a carrier has 1,000,000 live spores on it, a 6-log reduction would reduce the number of live spores to 1.

The term "sterilization" is used to refer to a process wherein there is a total absence of living test microorganisms on the biological indicator remaining after the sterilization process has been completed. However, processes that are less rigorous than sterilization processes including, for example, disinfection, sanitization, decontamination, cleaning processes, and the like, may be of value and are taken into account with this invention. Unless otherwise indicated, the term "sterilization" is used herein to refer to sterilization processes as well as less rigorous processes such as disinfection, sanitation, decontamination, cleaning, and the like.

The term "sterilant" refers to any medium that can be used to sterilize a substrate (e.g., a medical device, the interior of a room, etc.). The sterilant may comprise a liquid or a gas. In an embodiment, the sterilant comprises vaporous hydrogen peroxide, steam, ethylene oxide, peracetic acid, ozone, or a combination of two or more thereof.

The sterilization process provided for herein may employ any of the above-indicated sterilants. The sterilization process may be conducted for an effective period of time to achieve at least a 4 log reduction, or at least a 5 log reduction, or at least a 6 log reduction in the number of spores used as test microorganisms which are capable of reproduction, metabolism and/or growth. When at least a 6 log reduction is achieved, the process may be referred to as a sterilization process. When a 4 log reduction or a 5 log reduction is achieved, the process may be considered to be less rigorous than a sterilization process, but nevertheless useful for various disinfection, sanitization, decontamination and/or cleaning applications.

In an embodiment, the inoculum comprises water and the spores. The water may comprise tap water, deionized water, distilled water, water purified by osmosis, or a mixture of two or more thereof.

In an embodiment, the spores comprise bacterial spores. In an embodiment, the spores comprise spores of the *Bacillus* or *Clostridia* genera. In an embodiment, the spores comprise spores of *Geobacillus stearothermophilus, Bacillus atrophaeus, Bacillus sphaericus, Bacillus anthracis, Bacillus pumilus, Bacillus coagulans, Clostridium sporogenes, Clostridium difficile, Clostridium botulinum, Bacillus subtilis globigii, Bacillus cereus, Bacillus circulans,* or a mixture of two or more thereof. In an embodiment, the spores comprise spores of *Geobacillus stearothermophilus.* The concentration of spores in the inoculum may range from about 1x10³ to about 1x10⁶ colony-forming units (cfu) per microliter (µl), or from about 1x10⁴ to about 1x10⁶ cfu/µl.

In an embodiment, the carrier comprises a hydrophobic substrate. The carrier may comprise a non-porous material. The carrier may comprise a solid material. The carrier may comprise any material that does not dissolve or deteriorate during the sterilization or incubation processes. The carrier may comprise any material that does not combust with a high concentration of an oxidizing sterilant. The carrier may comprise metal, glass, ceramics, plastic, or a combination of two or more thereof. The metal may comprise aluminum or steel. The plastic may comprise a polyolefin, polystyrene, polycarbonate, polymethacrylate, polyacrylamide, polyimide, polyester, and the like. The carrier may comprise a film. The carrier may comprise a surface within a biological indicator, for example, a self-contained biological indicator (SCBI).

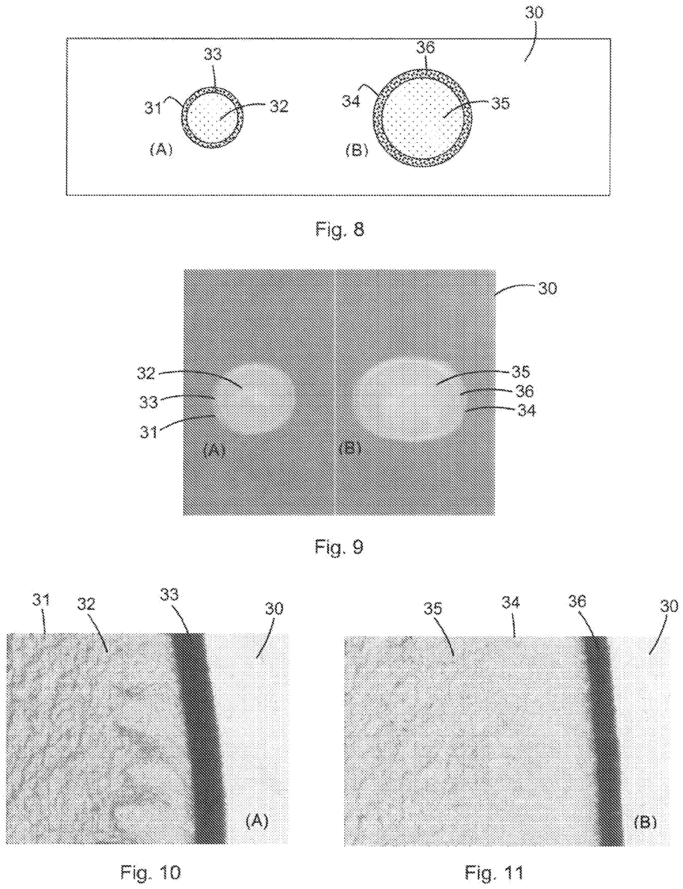

The spore deposit may have a central region and a peripheral region. See, for example, Fig. 8 wherein spore deposit 31 includes central region 32 and peripheral region 33. The spore deposit may separate into a central region and a peripheral region due to the fact that as the drop of inoculum dries, the spores tend to not distribute on the carrier in a uniform, monodispersed manner. Instead, as the drop dries, the spores tend to accumulate in the peripheral region of the spore deposit. As such, the concentration of spores in the peripheral region may be higher than in the central region. The number of spores in the spore deposit may be in the range from about 1x10⁶ to about 5x10⁷ cfu, or from about 1x10⁶ to about 5x10⁶ cfu. The number of spores in the central region of the spore deposit may be from about 0.5x10⁵ to about 2.5x10⁷ cfu, or from about 0.5x10⁶ to about 2.5x10⁶ cfu. The number of spores in the peripheral region of the spore deposit may be from about 0.5x10⁶ to about 2.5x10⁷ cfu, or from about 0.5x10⁶ to about 2.5x10⁶ cfu. The central region of the spore deposit may comprise a single spore layer with little or no spore stacking (for example, less than about 1% of the area of the central region comprising spores in a stacked configuration, or less than about 0.1% spore stacking). The peripheral region may contain spores wherein the spores reside in a stacked configuration. The central region may comprise from about 70% to about 95%, or from about 80% to about 95%, or from about 90% to about 95% of the area of the spore deposit. The peripheral region may comprise from about 5% to about 30%, or from about 5 to about 20%, or from about 5% to about 10% of the area of the spore deposit.

By increasing the volume of the inoculum, while holding the total number of spores deposited the same, it is possible to disproportionately increase the area of the central region with little or no stacking relative to the peripheral region with stacking.

The carrier may be positioned in an indicator device, for example, a self-contained biological indicator (SCBI). The carrier may comprise a surface in a compartment of an indicator device. The carrier may be inoculated with the inoculum. The biological indicator may comprise a SCBI with two separate compartments, one of which contains the inoculated carrier and the other of which contains a growth media. The growth media may be in a frangible glass vial that can be broken just prior to incubation. In use, the biological indicator and the article to be sterilized are exposed to a sterilant during a sterilization process. Then following sterilization, the biological indicator is activated to allow for the test microorganisms to come into contact with the growth media. The biological indicator may then be incubated to determine whether the sterilization process is effective.

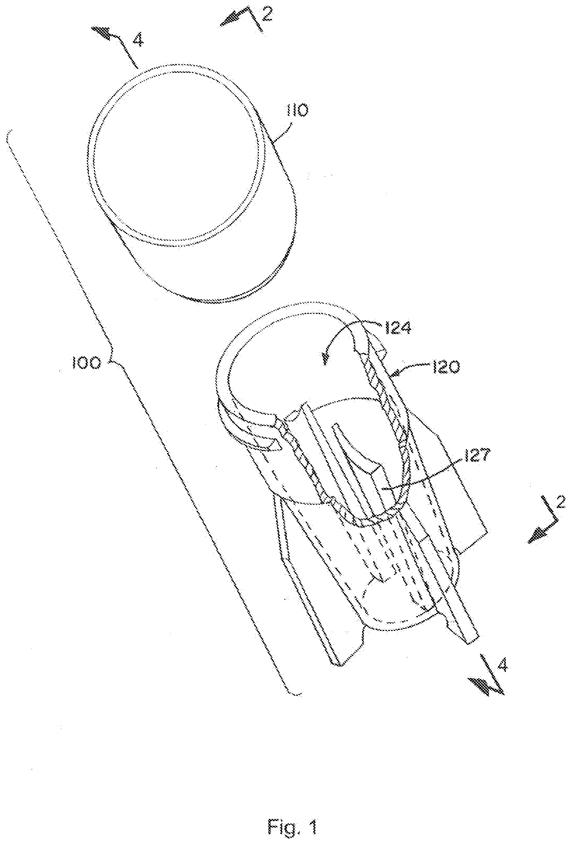

The indicator device may comprise a SCBI, which may be in the form illustrated in Figs. 1-4. Referring to Figs. 1-4, SCBI 100 includes cap 110 which is configured for housing fluid 140. The fluid 140 contains a growth media. Cap 110, which is mounted on container 120, includes inner chamber 116. The inner chamber 116 has an opening 115 with a breakable barrier 130 overlying the opening 115. The fluid 140 is encapsulated within the inner chamber 116. The container 120 has an interior region 124 where carrier 190 is positioned. The carrier 190 may be inoculated as described above to form a spore deposit on the carrier 190.

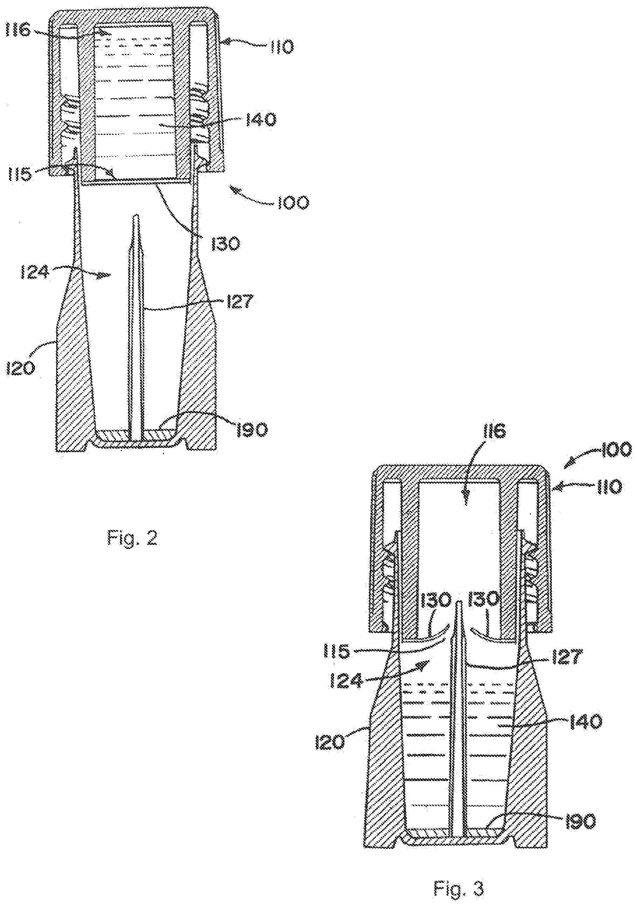

When used in a sterilization process, the cap 110 is held in an open position as illustrated in Fig. 2. The SCBI 100 and items to be sterilized are then subjected to the sterilization process. During the sterilization process, the sterilant passes through openings between the cap 110 and the container 120 and flows into the interior region 124 where it contacts and acts upon the test organism spores deposited on the carrier 190.

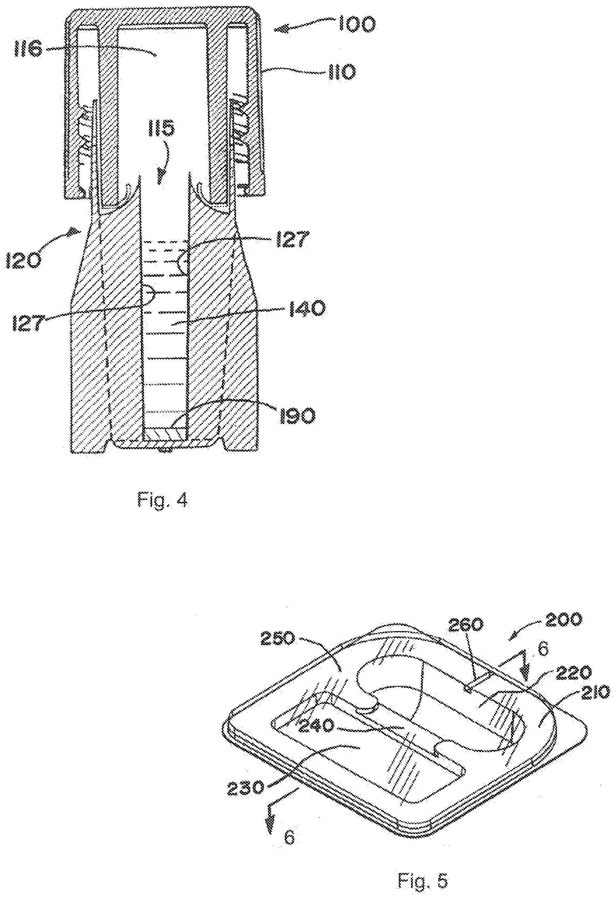

After the sterilization process is complete, the SCBI 100 is activated by screwing the cap 110 downward into a closed position as shown in Figs. 3 and 4. This results in the breakable barrier 115 being broken by puncture member 127 to form broken barrier 130. (Note that two puncture members may be used as shown in the embodiment illustrated in Fig. 4). Fluid 140, which contains a growth media, then flows into the container 120 in contact with the spores deposited on the carrier 190.

While in container 120, the spores and the growth media may be incubated for a sufficient period of time to determine the viability of the spores. At the end of the incubation period, the SCBI may be evaluated to determine whether any spores survive the sterilization process. If the spores survive the sterilization process, the sterilization process is not considered to have been successful. On the other hand, if the spores are killed, then the sterilization process is considered to be successful.

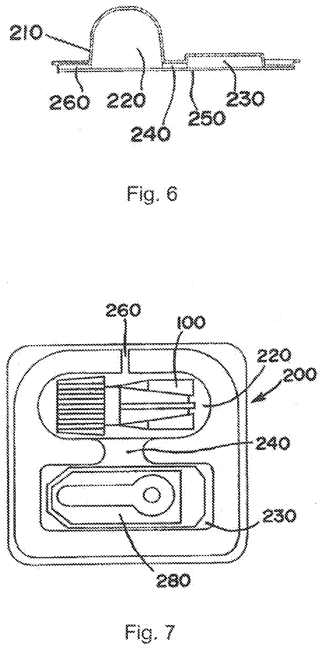

The SCBI 100 may be used with a test pack as depicted in Figs. 5-7. Referring to Figs. 5-7, test pack 200 includes base 210 containing recessed compartments 220 and 230. The recessed compartments 220 and 230 are in fluid communication with each other via internal channel 240. Cover 250 is attached to the base 210 and forms a sealed enclosure for the recessed compartments 220 and 230. External channel 260 provides a fluid communication between the sealed enclosure and an external environment. The SCBI 100 is positioned in recessed compartment 220. A chemical integrator and/or a chemical indicator 280 is positioned in recessed compartment 230. The external channel 260 is configured to allow a restricted flow of sterilant into the recessed compartments 220 and 230. The base 210 and cover 250 are otherwise impenetrable by the sterilant. The chemical integrator and/or chemical indicator 280 may undergo a change in color after it has been exposed to a sufficient quantity of sterilant in the sterilization medium for a sufficient period of time to indicate that the sterilization process has been completed. The sterilant also flows into the SCBI 100 in recessed compartment 220, and contacts the spores in the SCBI 100.

Another modification of the above forms of the biological indicator may be a rapid read or fast acting biological indicator. In this form the biological indicator may be mated to a dedicated instrument (reader) that detects early signals of spore viability.

The biological indicator may be used to release loads or validate sterilization chamber functionality in healthcare settings. The biological indicator may also be used to determine if biological indicator waste has been properly decontaminated. In the scientific setting, the biological indicator may be used to validate the functionality of sterilization chambers, release loads of goods, or validate that a process meets required functionality. A valid biological indicator for a given process requires a specific resistance and therefore biological indicator manufacturers may strive to manufacture the biological indicator with targeted resistance characteristics.

The biological indicator may be used by subjecting it to the same sterilization medium and treatment as the articles for which sterile conditions may be sought. Sterilant (e.g., vaporous hydrogen peroxide, ethylene oxide, steam, peracetic acid, ozone, etc.) may pass into the area where the biological indicator is located thereby exposing the biological indicator to the same sterilization process as the articles being sterilized. Following sterilization, growth media may be brought into contact with the biological indicator. The growth media may be in the form of a liquid. The growth media may comprise a buffered aqueous solution. Any procedure whereby the biological indicator is brought into contact with the growth media under conditions which allow for growth of live test microorganisms (i.e., spores), if any exists, may be used. The growth media may be present in the sterilization chamber in powder or tablet form and, after sterilization, sterile water may be added such that the biological indicator comes into contact with the aqueous incubation medium.

The growth media may comprise one or more nutrient sources. The nutrient source may be used to provide energy for the growth of any of the test organisms that may survive the sterilization process. Examples of the nutrient sources may include pancreatic digest of casein, enzymatic digest of soybean meal, sucrose, dextrose, yeast extract, L-cystine, and mixtures of two or more thereof.

A microbial growth indicator, which changes color or native state, in the presence of viable test organisms may be used with the growth media. The growth indicator may be dispersed or solubilized in the growth media and impart an initial color to the growth media. The growth indicator may also impart a color change in the growth media upon test organism growth. Growth indicators which may be employed include pH-sensitive dye indicators (such as bromothymol blue, bromocresol purple, phenol red, etc. or combinations thereof), oxidation-reduction dye indicators (such as methylene blue, etc.). The use of these microbial growth indicators may result in a change in color in response to a phenomenon of microorganism growth, such as changes in pH, oxidation-reduction potentials, enzymatic activity, as well as other indications of growth.

The growth media may further comprise one or more pH buffers, one or more neutralizers, one or more agents for maintaining osmotic equilibrium, or a mixture of two or more thereof.

The pH buffers may include K₂HPO₄, KH₂PO₄, (NH₄)₂HPO₄, 2,2-Bis(hydroxylmethyl)-2,2',2"-nitrilothiethanol (Bis Tris), 1, 3-Bis[tris(hydroxymethyl)methylamino] propane (Bis-Tris Propane), 4-(2-Hydroxyethyl)piperazine-ethanesulfonic acid (HEPES), 2-Amino-2-(hydroxymethyl)-1,3-propanediol (Trizma, Tris base), N-[Tris(hydroxymethyl)methyl]glycine (Tricine), Diglycine (Gly-Gly), N,N-Bis(2-hydroxyethyl)glycine (Bicine), N-(2-Acetamido)iminodiacetic acid (ADA), N-(2-Acetamido)-2-aminoethanesulfonic acid (aces), 1,4-Piperazinediethanesulfonic acid (PIPES), Beta-Hydroxy-4-morpholinepropanesulfonic acid (MOPSO), N,N-Bis(2-hydroxyethyl)-2-aminoethanesulfonic acid (BES), 3-(N-Morpholino)propanesulfonic acid (MOPS), 2-[(2-Hydroxy-1,1-bis(hydroxylmethyl)ethyl)amino]ethanesulfonic acid (TES), 3-(N,N-Bis[2-hydroxyethyl]amino)-2-hydroxypropanesulfonic acid (DIPSO), 4-(N-Morpholino)butanesulfonic acid (MOBS), 2-Hydroxy-3-[tris(hydroxymethyl)methylamino]-1-propanesulfonic acid (TAPSO), 4-(2-Hydroxyethyl)piperazine-1-(2-hydroxypropanesulfonic acid hydrate (HEPPSO), Piperazine-1,4-bis(2-hydroxypropanesulfonic acid) dihydrate (POPSO), 4-(2-Hydroxyethyl)-1-piperazine propanesulfonic acid (EPPS), N-(2-Hydroxyethyl)piperazine-N'-(4-butanesulfonic acid) (HEPBS), [(2-Hydroxy-1,1-bis(hydroxymethyl)ethyl)amino]-1-propanesulfonic acid (TAPS), 2-Amino-2-methyl-1,3-propanediol (AMPD), N-tris(Hydroxymethyl)methyl-4-aminobutanesulfonic acid (TABS), N-(1,1-Dimethyl-2-hydroxyethyl)-3-amino-2-hydroxypropanesulfonic acid (AMPSO), 2-(Cyclohexylamino)ethanesulfonic acid (CHES), 3-(Cyclohexylamino)-2-hydroxyl-1-propanesulfonic acid (CAPSO), 2-Amino-2-methyl-1-propanol (AMP), 3-(Cyclohexylamino)-1-propanesulfonic acid (CAPS), 4-(Cyclohexylamino)-1-butanesulfonic acid (CABS), 2-(N-Morpholino)ethanesulfonic acid hydrate (MES), N-(2-Acetamido)-2-aminoethanesulfonic acid (ACES), and mixtures of two or more thereof.

The neutralizers may include but are not limited to sodium thioglycollate, sodium thiosulfate, catalase, sodium bisulfate, sodium bisulfite lecithin, polysorbate 20, polysorbate 80, calcium bicarbonate, and mixtures of two or more thereof.

The agents for maintaining osmotic equilibrium may include sodium salt, potassium salts, magnesium salts, manganese salts, calcium salts, metallic salts, sodium chloride, potassium chloride, magnesium sulfate, iron chloride, and mixtures of two or more thereof.

The growth media may comprise an aqueous composition comprising: water; from about 0.01 to about 100 grams per liter (g/l), or from about 0.1 to about 50 g/l, of one or more nutrient sources; from about 1.0x10-5 to about 10 g/l, or from about 1.0x10-4 to about 1.0 g/l of one or more microbial growth indicators; up to about 5000 g/l, or from about 0.001 to about 5000 g/l, or from about 0.1 to about 1000 g/l, of one or more pH buffers; up to about 100 g/l, or from about 0.01 to about 100 g/l, or from about 0.1 to about 50 g/l, of one or more neutralizers; up to about 50 g/l, or from about 0.1 to about 50 g/l, or from about 0.1 to about 25 g/l, of one or more agents for maintaining osmotic equilibrium.

### Examples

### Example 1

Spores of *Geobacillus stearothermophilus* are suspended in water to form an inoculum. The concentration of spores in the inoculum is either (A) 1x10⁵ cfu per microliter or (B) 0.5x10⁵ cfu per microliter. A 20 microliter drop of (A) and a 40 microliter drop of (B) are deposited on a glass slide. The same number of spores (2x10⁶) are deposited with each of (A) and (B). The surface area at the base of the deposited drop increases from 0.141 cm² for drop (A) to 0.224 cm² for drop (B), i.e., an increase of 59%. This is shown in Table 1. The circumference of the deposited drops increases from 1.333 cm for drop (A) to 1.679 cm for drop (B), an increase of 26%. This is shown in Table 2. The ratio of circumference to surface area decreases from 9.454 for drop (A) to 7.496 for drop (B), a decrease of 21%. This is shown in Table 3.

**Table 1: Surface areas of 20 v. 40 microliter drops**

| 20 microliter drop (A) | 40 microliter drop (B) | Increase in area |
|---|---|---|
| 0.141 cm² | 0.224 cm² | 59%* |

| | | |
|---|---|---|
| *(0.224-0.141 =0.083)/0.141 x 100 = 59% | | |

**Table 2: Circumferences of 20 v. 40 microliter drops**

| 20 microliter droplet (A) | 40 microliter droplet (B) | Increase in circumference |
|---|---|---|
| 1.333 cm | 1.679 cm | 26%* |

| | | |
|---|---|---|
| *(1.679-1.333=0.346)/1.333 x 100 = 26% | | |

**Table 3: Ratio of circumference to surface area of 20 v. 40 microliter drops**

| 20 microliter droplet (A) | 40 microliter droplet (B) | Decrease in ratio |
|---|---|---|
| 9.454 | 7.496 | 21% |

These results indicate that by increasing the volume of the inocula from 20 to 40 microliters, the area of the deposit with the monodispersed spores (i.e., spores residing in a single spore layer) increases more than the non-monodispersed peripheral region or circumference where stacking occurs.

### Example 2

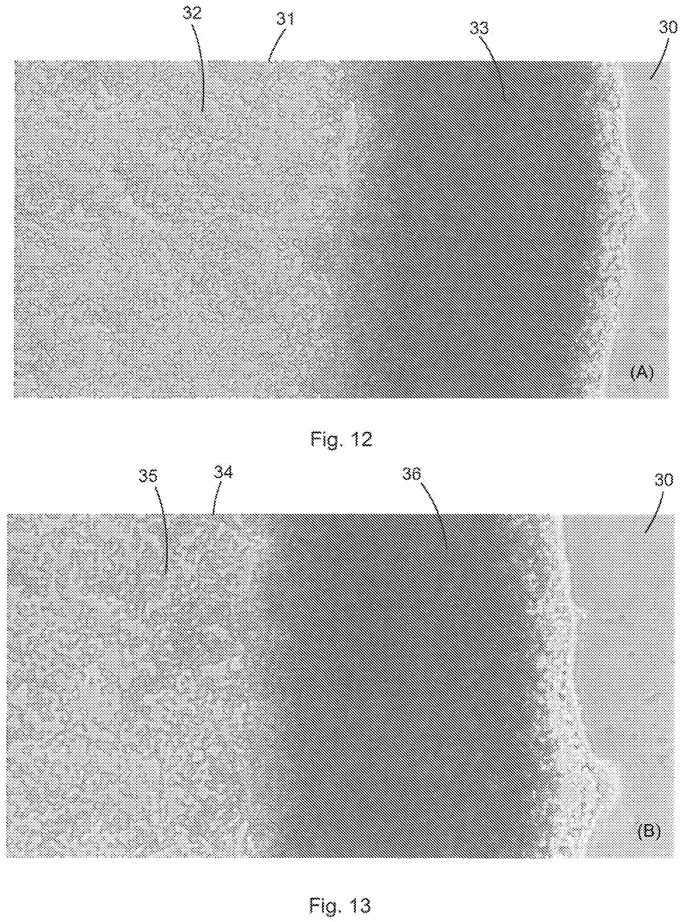

The drops (A) and (B) from Example 1 are dried to form spore deposits. The spore deposits are shown in Figs. 8-13. Referring to Figs. 8-13, spore deposit 31 from drop (A) is formed on glass slide 30. Spore deposit 31 includes central region 32 and peripheral region 33. Similarly, spore deposit 34 from drop (B) is formed on glass slide 30. Spore deposit 34 includes central region 35 and peripheral region 36. As the drops dry, the spores do not distribute on the glass slide 30 in a uniform, monodispersed manner (i.e., single spore layer). Instead, as the drops dry, the spores tend to accumulate in the peripheral regions 33 and 36 of spore deposits. This has the effect of stacking the spores in the peripheral regions 33 and 36. The resulting configuration of stacked spores in the peripheral regions 33 and 36 can be referred to as a "coffee-ring" effect. For the spore deposit 31, 80% of the area of the spore deposit comprises spores that reside in a single spore layer, the single spore layer being in central region 32. For the spore deposit 34, 83% of the area of the spore deposit comprises spores that reside in a single spore layer, the single spore layer being in central region 35.

### Example 3

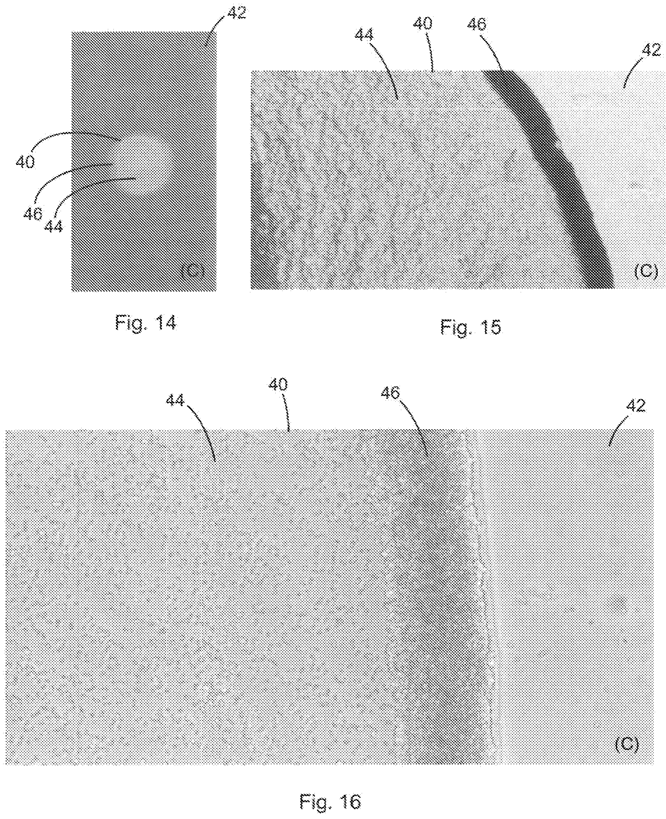

The spores from Example 1 are suspended in water to form an inoculum. The concentration of spores in the inoculum is 1x10⁵ cfu per microliter. A 20 microliter drop (A) and two 10 microliter drops (C) are deposited on a glass plate. The 20 microliter drop (A) corresponds to drop (A) in Example 1. One of the spore deposits from drop (C) is shown in Figs. 14-16. Referring to Figs. 14-16, spore deposit 40 is deposited on glass slide 42 and includes central region 44 and peripheral region 46. As indicated above, drop (A) has a surface area at the base of the deposited drop of 0.141 cm² and a circumference of 1.333 cm. Each of the two 10 microliter drops (C) has a surface area at the base of the deposited droplet of 0.089 cm² and a circumference of 1.058 cm. As shown in Table 4, the use of two 10 microliter drops (C) as compared to a single 20 microliter drop (A) increases the surface area at the base of the drops by 26%. As shown in Table 5, the circumference of one 20 microliter drop (A) is 1.333 cm, while the combined circumferences of two 10 microliter drops (C) is 2.116 cm for an increase in total circumference of 59%. This is more than half the increase of using the single 40 microliter drop (B) (1.679 cm) shown in Table 2. As shown in Table 6, the ratio of circumferences to surface area of one 20 microliter drop (A) is 9.454, while the combined ratio of two 10 microliter drops (C) is 11.888 for an increase in ratio of 26%.

**Table 4: Surface area of single 20 microliter drop (A) compared to two 10 microliter drops (C)**

| 20 microliter drop (A) | 2 x 10 microliter drop (C) | Increase in area |
|---|---|---|
| 0.141 cm² | 2 x 0.089 cm² = 0.178 cm² | 26%* |

| | | |
|---|---|---|
| *(0.178-0.141=0.037)/0.141 x 100 = 26% | | |

**Table 5: Circumferences of single 20 microliter drop (A) compared to two 10 microliter drops (C)**

| 20 microliter drop (A) | 2 x 10 microliter drops (C) | Increase in circumference |
|---|---|---|
| 1.333 cm | 2 x 1.058 = 2.116 cm | 59%* |

| | | |
|---|---|---|
| *(2.116-1.333=0.783)/1.333 x 100 = 59% | | |

**Table 6: Ratio of circumference to surface area of single 20 microliter drop (A) compared to two 10 microliter drops (C)**

| 20 microliter drop (A) | 2 x 10 microliter drops (C) | Increase in ratio |
|---|---|---|
| 9.454 | 11.888 | 26% |

By distributing the same inocula across two separate deposits, the resulting combined circumference exceeds the combined increase in the area achieved. This means that it is possible to control the ratio of circumference to surface area to make adjustments in the proportions of spores that are monodisperse (i.e., single spore layer) versus stacked. This can be used to modify the required exposure times for the inventive biological indicator to achieve different partial and all kill times.

While the invention has been explained in relation to various embodiments, it is to be understood that various modifications thereof will become apparent to those skilled in the art upon reading the specification.

## Claims

1. A biological indicator, comprising:
a carrier; and
a spore deposit on the carrier, wherein from about 70% to about 95% of the area of the spore deposit comprises spores residing in a single spore layer, and the remainder of the area of the spore deposit comprises spores residing in a stacked configuration; and
wherein the biological indicator is in a first compartment of an indicator device, the first compartment being adapted to permit the spores to be brought into contact with a sterilant during a sterilization process; and the indicator device further comprises a second compartment containing a growth media, the second compartment being adapted to maintain the growth media separate from the spores during the sterilization process, and to permit the growth media to contact the spores after the sterilization process is completed.

2. The biological indicator of claim 1 wherein the spore deposit is formed by the deposition of an inoculum on the carrier, the inoculum comprising water and spores dispersed in the water, the inoculum being dried on the carrier to form the spore deposit.

3. The biological indicator of claim 2 wherein the inoculum forms a perimeter upon being deposited on the carrier, and prior to being dried the inoculum is spread to extend the perimeter.

4. The biological indicator of any one of the preceding claims 1-3 wherein the carrier comprises a hydrophobic substrate.

5. The biological indicator of any one of the preceding claims 2-4 wherein the inoculum deposited on the carrier has a volume in the range from about 10 to about 1000 microliters.

6. The biological indicator of claim 1 wherein the indicator device is positioned in a test pack.

7. The biological indicator of any one of the preceding claims 1-6 wherein the spores comprise bacterial spores.

8. The biological indicator of any one of the preceding claims 1-7 wherein the spores comprise spores of the *Bacillus or Clostridia* genera.

9. The biological indicator of any one of the preceding claims 1-8 wherein the spores comprise spores of *Geobacillus stearothermophilus, Bacillus atrophaeus, Bacillus sphaericus, Bacillus anthracis, Bacillus pumilus, Bacillus coagulans, Clostridium sporogenes, Clostridium difficile, Clostridium botulinum, Bacillus subtilis globigii, Bacillus cereus, Bacillus circulans,* or a mixture of two or more thereof.

10. The biological indicator of any one of the preceding claims 1-9 wherein the spores comprise *Geobacillus stearothermophilus* spores.

11. A sterilization process, comprising: exposing an article to be sterilized and the biological indicator of any one of the preceding claims 1-10 to a sterilant.

12. The process of claim 11 wherein the sterilant comprises vaporous hydrogen peroxide, ethylene oxide, steam, peracetic acid, ozone, or a combination of two or more thereof.

13. A process for determining the effectiveness of a sterilization process, comprising:
exposing an article to be sterilized and the biological indicator of any one of claims 1-10 to a sterilant; and
incubating the biological indicator in the presence of a growth media to determine whether the sterilization process is effective.

## Patentansprüche

1. Biologischer Indikator, umfassend:
einen Träger; und
eine Sporenablagerung auf dem Träger, wobei etwa 70 % bis etwa 95 % der Fläche der Sporenablagerung Sporen umfasst, die in einer einzelnen Sporenschicht liegen, und der Rest der Fläche der Sporenablagerung Sporen umfasst, die in einer gestapelten Konfiguration liegen; und
wobei sich der biologische Indikator in einem ersten Kompartiment einer Indikatorvorrichtung befindet, wobei das erste Kompartiment derart beschaffen ist, dass die Sporen während eines Sterilisationsprozesses in Kontakt mit einem Sterilisationsmittel gebracht werden können; und die Indikatorvorrichtung ferner ein zweites Kompartiment umfasst, das ein Wachstumsmedium enthält, wobei das zweite Kompartiment derart beschaffen ist, dass das Wachstumsmedium während des Sterilisationsprozesses von den Sporen getrennt gehalten wird und dass das Wachstumsmedium nach Abschluss des Sterilisationsprozesses mit den Sporen in Kontakt kommen kann.

2. Biologischer Indikator nach Anspruch 1, wobei die Sporenablagerung durch die Ablagerung eines Inokulums auf dem Träger gebildet wird, wobei das Inokulum Wasser und in dem Wasser dispergierte Sporen umfasst, wobei das Inokulum auf dem Träger getrocknet wird, um die Sporenablagerung zu bilden.

3. Biologischer Indikator nach Anspruch 2, wobei das Inokulum nach dem Ablagern auf dem Träger einen Umfang bildet und das Inokulum vor dem Trocknen ausgebreitet wird, um den Umfang zu vergrößern.

4. Biologischer Indikator nach einem der vorhergehenden Ansprüche 1-3, wobei der Träger ein hydrophobes Substrat umfasst.

5. Biologischer Indikator nach einem der vorhergehenden Ansprüche 2-4, wobei das auf dem Träger abgelagerte Inokulum ein Volumen im Bereich von etwa 10 bis etwa 1000 Mikrolitern aufweist.

6. Biologischer Indikator nach Anspruch 1, wobei die Indikatorvorrichtung in einer Testpackung positioniert ist.

7. Biologischer Indikator nach einem der vorhergehenden Ansprüche 1-6, wobei die Sporen bakterielle Sporen umfassen.

8. Biologischer Indikator nach einem der vorhergehenden Ansprüche 1-7, wobei die Sporen Sporen der Gattungen *Bacillus* oder *Clostridium* umfassen.

9. Biologischer Indikator nach einem der vorhergehenden Ansprüche 1-8, wobei die Sporen Sporen von *Geobacillus stearothermophilus, Bacillus atrophaeus, Bacillus sphaericus, Bacillus anthracis, Bacillus pumilus, Bacillus coagulans, Clostridium sporogenes, Clostridium difficile, Clostridium botulinum, Bacillus subtilis globigii, Bacillus cereus, Bacillus circulans* oder ein Gemisch aus zwei oder mehreren davon umfassen.

10. Biologischer Indikator nach einem der vorhergehenden Ansprüche 1-9, wobei die Sporen Sporen von *Geobacillus stearothermophilus* umfassen.

11. Sterilisationsverfahren, umfassend: Aussetzen eines zu sterilisierenden Gegenstands und des biologischen Indikators nach einem der vorhergehenden Ansprüche 1-10 einem Sterilisationsmittel.

12. Verfahren nach Anspruch 11, wobei das Sterilisationsmittel dampfförmiges Wasserstoffperoxid, Ethylenoxid, Dampf, Peressigsäure, Ozon oder eine Kombination von zwei oder mehreren davon umfasst.

13. Verfahren zum Bestimmen der Wirksamkeit eines Sterilisationsverfahrens, umfassend:
Aussetzen eines zu sterilisierenden Gegenstands und des biologischen Indikators nach einem der Ansprüche 1-10 einem Sterilisationsmittel; und
Inkubieren des biologischen Indikators in Gegenwart eines Wachstumsmediums zum Bestimmen, ob das Sterilisationsverfahren wirksam ist.

## Revendications

1. Indicateur biologique, comprenant :
un support ; et
un dépôt de spores sur le support, dans lequel environ 70 % à environ 95 % de la surface du dépôt de spores comprend des spores résidant dans une seule couche de spores, et le reste de la surface du dépôt de spores comprend des spores résidant dans une configuration empilée ; et
dans lequel l'indicateur biologique est dans un premier compartiment d'un dispositif indicateur, le premier compartiment étant adapté pour permettre aux spores d'être mises en contact avec un stérilisant pendant un processus de stérilisation ; et le dispositif indicateur comprend également un second compartiment contenant un milieu de croissance, le second compartiment étant adapté pour maintenir le milieu de croissance séparé des spores pendant le processus de stérilisation, et pour permettre au milieu de croissance d'entrer en contact avec les spores une fois le processus de stérilisation terminé.

2. Indicateur biologique selon la revendication 1, dans lequel le dépôt de spores est formé par le dépôt d'un inoculum sur le support, l'inoculum comprenant de l'eau et des spores dispersées dans l'eau, l'inoculum étant séché sur le support pour former le dépôt de spores.

3. Indicateur biologique selon la revendication 2 dans lequel l'inoculum forme un périmètre lorsqu'il est déposé sur le support, et avant d'être séché l'inoculum est étalé pour étendre le périmètre.

4. Indicateur biologique selon l'une quelconque des revendications précédentes 1 à 3 dans lequel le support comprend un substrat hydrophobe.

5. Indicateur biologique selon l'une quelconque des revendications précédentes 2 à 4 dans lequel l'inoculum déposé sur le support a un volume dans la plage d'environ 10 à environ 1 000 microlitres.

6. Indicateur biologique selon la revendication 1 dans lequel le dispositif indicateur est positionné dans un pack de test.

7. Indicateur biologique selon l'une quelconque des revendications précédentes 1 à 6 dans lequel les spores comprennent des spores bactériennes.

8. Indicateur biologique selon l'une quelconque des revendications précédentes 1 à 7 dans lequel les spores comprennent des spores des genres *Bacillus* ou *Clostridia.*

9. Indicateur biologique selon l'une quelconque des revendications précédentes 1 à 8 dans lequel les spores comprennent des spores de *Geobacillus stearothermophilus, Bacillus atrophaeus, Bacillus sphaericus, Bacillus anthracis, Bacillus pumilus, Bacillus coagulans, Clostridium sporogenes, Clostridium difficile, Clostridium botulinum, Bacillus subtilis globigii, Bacillus cereus, Bacillus circulans,* ou un mélange de deux ou plusieurs de ceux-ci.

10. Indicateur biologique selon l'une quelconque des revendications précédentes 1 à 9 dans lequel les spores comprennent des spores de *Geobacillus stearothermophilus.*

11. Procédé de stérilisation, comprenant : l'exposition d'un article à stériliser et de l'indicateur biologique selon l'une quelconque des revendications précédentes 1 à 10 à un stérilisant.

12. Procédé selon la revendication 11 dans lequel le stérilisant comprend du peroxyde d'hydrogène vaporeux, de l'oxyde d'éthylène, de la vapeur d'eau, de l'acide peracétique, de l'ozone, ou une combinaison de deux ou plusieurs de ceux-ci.

13. Procédé de détermination de l'efficacité d'un processus de stérilisation, comprenant :
l'exposition d'un article à stériliser et de l'indicateur biologique selon l'une quelconque des revendications précédentes 1 à 10 à un stérilisant ; et
l'incubation de l'indicateur biologique en présence d'un milieu de croissance pour déterminer si le processus de stérilisation est efficace.
